# EUROPEAN PATENT APPLICATION

(11) **EP 2 578 102 A1**
(43) Date of publication of application: **10.04.2013**
(21) Application number: 12165812.4
(22) Date of filing: 26.04.2012
(51) Int. Cl.: A43D 999/00, G01N 3/56

(54) **Machine for carrying out abrasion tests on shoe soles**

(30) Priority: 05.10.2011 IT VR20110190
(71) Applicant: Vibram S.p.A., 21041 Albizzate (Varese) (IT)
(72) Inventor: Bramani, Marco, 21041 Albizzate (Varese) (IT); Sacchi, Massimiliano, 21041 Albizzate (Varese) (IT)
(74) Representative: Feltrinelli, Secondo Andrea

(57) **Abstract**

A machine (1) for carrying out abrasion tests on materials for shoe soles or on parts of soles or on complete soles comprises a frame (2) with a specimen holder (3, 300), a specimen (4, 400) fixed to the specimen holder (3, 300), an abrasive surface (5), a mobile base block (6) for supporting the abrasive surface (5), the mobile base block (6) being connected to the frame (2), a first cyclical movement mechanism (7) of the mobile support base (6), a second cyclical movement mechanism (8) of the specimen holder (3) towards the abrasive surface (5), the second movement mechanism (8) includes a tie rod of variable length (9) connected to the frame (2) with an actuator (10) to press and allow variable inclinations of the specimen (4, 400) against the abrasive surface (5), and a cleaning device (11) of the abrasive surface (5).

## Description

### TECHNICAL FIELD OF THE INVENTION

The present invention refers to a machine for carrying out abrasion tests in particular on materials used in making a shoe sole.

### STATE OF THE ART

Currently, in order to determine the resistance to abrasion of materials used in making a shoe sole lifetime tests are carried out by workers who wear shoes, with the sole to be tested, for long test periods. Although this method simulates the real conditions of use of shoe soles, it requires long testing periods, allowing only a finished product to be tested, with consequent high prototyping costs, and also introducing a high degree of uncertainty in the results, due to the subjectivity of execution of the test.

On the market there are also machines for performing abrasion tests with shorter analysis times. Such machines make it possible to determine the resistance to abrasion of a specimen, suitably shaped, placed in contact with an abrasive material, in relative motion with respect to the specimen.

In some embodiments, the abraded material can be partially removed by the abrasive surface during the test itself.

However, these machines do have limitations, since they allow only some types of abrasive surface to be used and the cleaning of the testing surface is, generally, only partial.

Moreover, it is not possible to analyse the wear of a complete sole, or of a possible tread thereof, being limited to performing an abrasion test of just the typical shape of the specimen.

Finally, it is not possible to vary the angle of incidence of the specimen against the abrasive surface or the force with which the specimen itself is kept in contact against the surface, to simulate different abrasion and wear conditions to which the material that constitutes the shoe soles is normally subjected.

### PURPOSES OF THE INVENTION

A purpose of the present invention is to improve the state of the art.

Another purpose of the present invention is to propose a machine for performing abrasion tests that makes it possible to determine the resistance to wear of materials, in particular of the materials used in making a shoe sole, through the cyclical movement of a specimen against an abrasive surface set in relative motion with respect to the specimen itself. Another purpose of the present invention is to propose a machine for carrying out abrasion tests that allows the abrasive surface to be kept clean during the test, in order to keep the execution conditions of the test stable, in particular the friction coefficient of the abrasive surface.

In accordance with an aspect of the present invention a machine according to claim 1 is foreseen.

The dependent claims refer to preferred and advantageous embodiments of the invention.

### BRIEF DESCRIPTION OF THE DRAWINGS

Further characteristics and advantages of the present invention will become clearer from the detailed description of a preferred, but not exclusive, embodiment of a machine for carrying out abrasion tests, in particular on materials used in making a shoe sole, illustrated for indicating but not limiting purposes, in the attached tables of drawings, in which:
figure 1 is a side view of the machine;
figure 2 is a front view of the machine;
figure 3 is a perspective view of a detail of the machine;
figure 4 is a front view of the detail of figure 3;
figure 5 is a perspective view of a detail of the machine;
figure 6 is a view from above of the detail of figure 5;
figure 7 is a side view of a detail of the machine;
figure 8 is an operating diagram of the machine;
figure 9 is a diagram relative to the operation of the machine;
figure 10 is a section view of a detail of figure 3;

### EMBODIMENTS OF THE INVENTION

With reference to the attached figures, reference numeral 1 schematically indicates a machine for carrying out abrasion tests of materials, in particular of the materials used in making shoe soles.

The machine 1 carries out a cyclical movement of a specimen against an abrasive surface set in relative motion with respect to the specimen itself, thus causing rubbing between the specimen and the abrasive surface itself.

Moreover, it is possible to test the resistance to abrasion not only of a suitably shaped test material but also of a complete sole, thus evaluating the resistance to abrasion of its possible tread.

The machine 1 also allows the abrasive surface to be kept constantly clean during the test through brushes, and thus allows the friction coefficient of the abrasive surface itself to be kept constant.

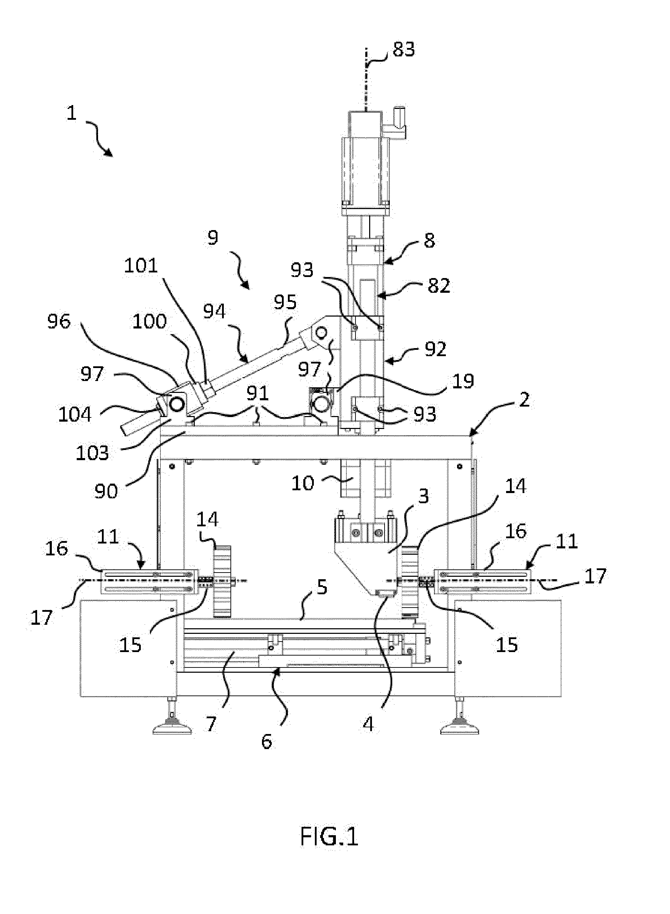

In reference to the embodiment of figure 1, the machine 1 comprises a frame 2 with which are associated a specimen holder 3, at least one specimen 4 fixed to the specimen holder, an abrasive surface 5, a mobile support base 6 of the abrasive surface 5, first cyclical movement means 7 of the mobile support base 6, second cyclical movement means 8 of the specimen holder 3 towards the abrasive surface 5, a tie rod of variable length 9 connected to the second movement means 8 and to the frame 2, at least one actuator 10, to press the specimen 4 against the abrasive surface 5, and at least one cleaning device 11 of the abrasive surface 5.

The second movement means 8 carry out a cyclical movement of the specimen 4 between two limit positions, a first starting position, in which the specimen 4 is not placed in contact with the abrasive surface 5, and a second end position, in which the specimen 4 is placed in contact with the abrasive surface 5.

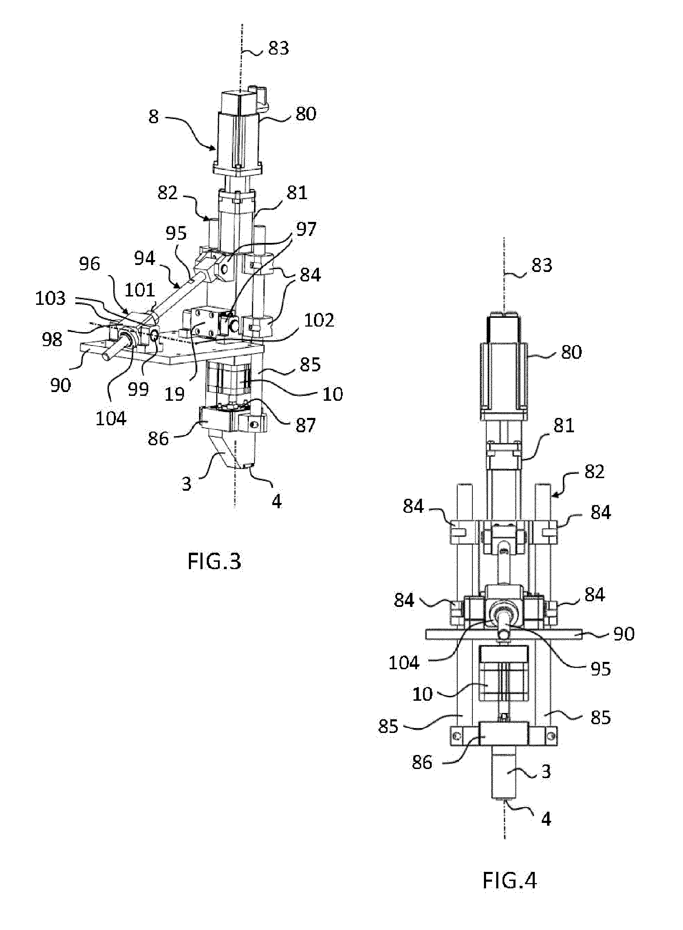

The second movement means 8, in accordance with the embodiment of figures 3 and 4, comprise motor means 80, a second motion transmission device 81, and second guiding means 82 of the specimen holder 3.

The second motor means 80, for example made with a gear reducer group with electric servomotor, cyclically actuate the second motion transmission device 81, for example a recirculating ball piston, or a rack gear, or a similar element, moving the specimen holder 3 along a second axis 83.

The actuator 10 allows a variable load force to be applied to the specimen 4, since the specimen is placed in contact with the abrasive surface 5, to simulate the loads to which the shoe soles are subjected for example while walking or while running. The actuator 10, which could be made through a single or double-acting pneumatic actuator, or an electric actuator or an element equipped with a compression spring or a similar device, is connected at one end to the second movement means 8, and at another end to a connection means 86 through an elastic joint 87. The elastic joint 87 makes it possible to reduce the stresses on the second movement means 8 caused by collisions and by the friction forces of the specimen 4 against the abrasive surface 5, while the abrasion test is being performed.

As shown in figures 3 and 4, the second guiding means 82, comprise at least one pair of sliding supports 84, for example self-lubricating bushings and/or sliding bushings and/or sleeves with balls or similar elements, at least one pair of tubular elements 85 each passing through a respective support 84, and a connection means 86 to constrain the specimen holder 3 to the second guiding means 82.

The second guiding means 82 make the movement of the specimen holder 3 along the axis 83 more stable while the abrasion test is being carried out.

In particular, the second guiding means 82 make it possible to reduce the extent of the collisions and of the cyclical stresses to which the second movement means 8 are subjected, due to the contact and the subsequent rubbing of the specimen 4 against the abrasive surface 5.

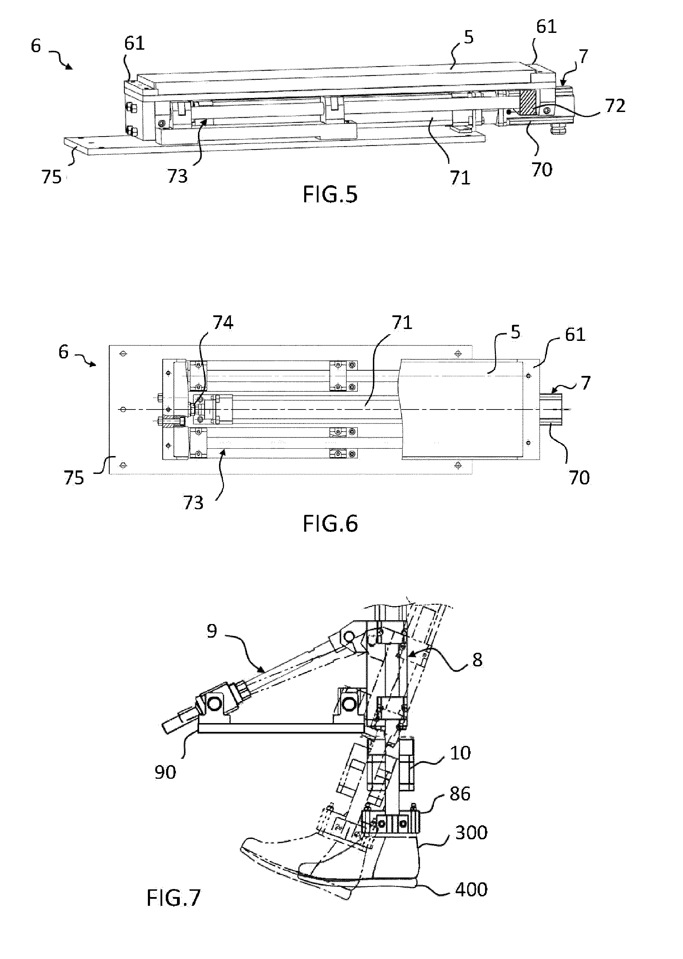

As shown in figures 5 and 6, the mobile support base 6 comprises at least one fixing element 61 of the abrasive surface 5 to the first movement means 7, and a second fixing element 75 to the frame 2.

The first movement means 7 make it possible to cyclically move the mobile support base 6, and therefore the abrasive surface 5, within two extreme positions - an initial starting position and a final end position - along a first guide 73.

The first movement means 7 comprise first motor means 70, for example a gear reducer group with electric motor or similar, a first device of transmission of the movement 71, for example a recirculating ball piston, and at least one end of stroke sensor 72 to determine when the end limit position of the mobile support base 6 along the first guide 73 has been reached.

The end of stroke sensor 72 could be made, for example, through a switch-operated sensor equipped with a flexible shaft and/or a probe sensor or similar.

The first movement means 7 are connected to the mobile support base 6 through a first elastic joint 74.

Similarly to what has been seen earlier relative to the second elastic joint 87, the first elastic joint 74 makes it possible to reduce the stresses on the first movement means 7 caused by the collisions and by the friction forces of the specimen 4 against the abrasive surface 5, and transmitted to the mobile support base 6, while the abrasion test is being carried out.

In an alternative embodiment of the first movement means 7, not depicted in the figures, the first motion transmission device 71 is, for example, made through a mat coated with abrasive material wound on rollers moved by the second motor means 70.

The fixing element 61, for example a plate constrained to the mobile support 6 through screws or similar, allows the abrasive surface 5 to be blocked to the mobile support base 6.

Moreover, by removing the fixing element 61 a rapid replacement of the abrasive surface 5 itself is ensured, when necessary.

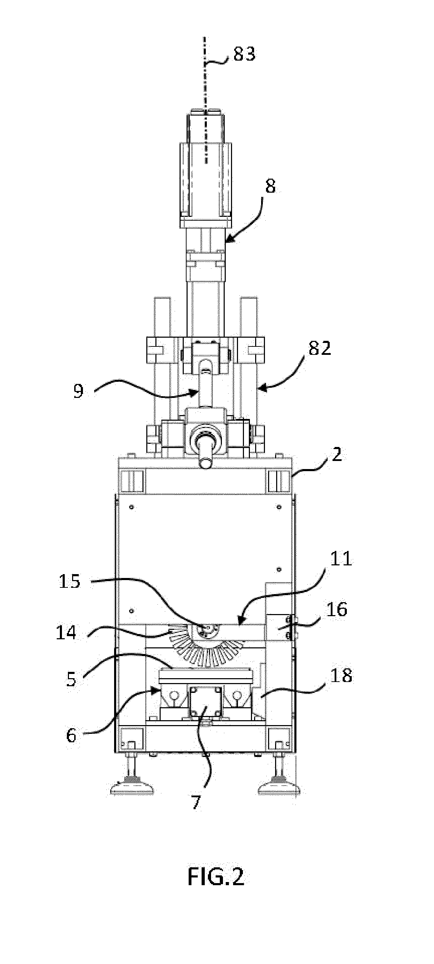

As shown in figures 1 and 2, the cleaning device 11 comprises at least one brush 14, third motor means 15 of the brush 14 and third connection means 16, for example an angular bracket, of the cleaning device 11 to the frame 2.

The cleaning device 11 makes it possible to clean the abrasive surface 5 while the test is being carried out and therefore to keep the friction coefficient of the abrasive surface 5 itself constant.

The brush 14 is placed in contact against the abrasive surface 5 and, while the test is being carried out, it is set in rotation by the third motor means 15 with respect to a first axis 17 parallel to the abrasive surface 5.

In a further embodiment of the cleaning device, the rotation axis of the brush 14 could be arranged perpendicular with respect to the abrasive surface 5. The brush 14 makes it possible to remove possible debris or material of the specimen 4 deposited during the test from the abrasive surface 5.

Moreover, the direction of rotation of the brush 14 allows such deposits to be taken away in the direction of a collection container 18 installed on the frame 2 near to the abrasive surface 5, as can be seen in figure 2.

The collection container 18 prevents possible debris from being dispersed into the installation area of the machine 1, thus allowing the area in which the test is carried out to be kept clean.

According to the embodiment of figures 1 and 3, the tie rod of variable length 9 forms part of a triangular-shaped structure in which two portions have a constant length, whereas a third portion that, indeed, is the tie rod 9 has a variable length.

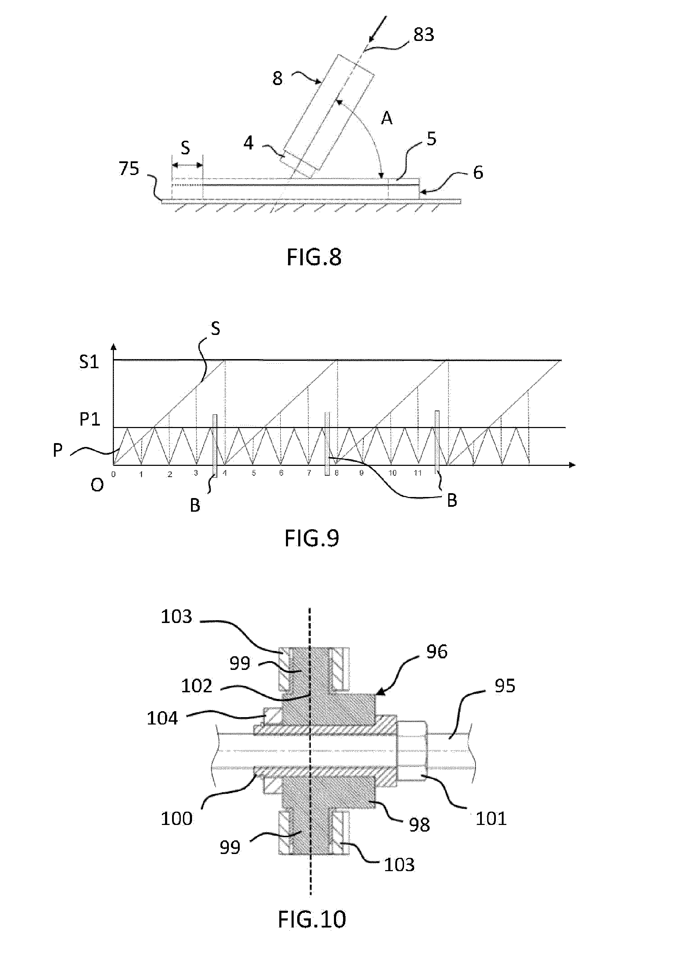

The tie rod of variable length 9 is connected to the others of the structure through rotary connection 97. According to what is illustrated in figure 8, the tie rod of variable length 9 makes it possible to vary the angle of inclination A of the second axis 83 with respect to the abrasive surface 5, in a range comprised between 60° and 90°.

The angle A also corresponds to the angle of incidence with which the specimen 4 comes into contact with the abrasive surface 5.

As shown in figure 1, a first portion 90, of fixed length, of the triangular-shaped structure is for example made from a horizontal flat plate.

The first portion 90 is fixed to the frame 2, through first fastening means 91, for example pins and/or bolts with relative nuts and/or similar.

A second portion 92, of fixed length, comprises second fastening means 93, for example pins and/or screws and/or similar elements, suitable for connecting the tie rod of variable length 9 to the second movement means 8 and to the second guiding means 82.

A third portion 94, of variable length, comprises a rod-shaped element 95 and at least one sleeve 96.

The sleeve 96 is constrained in a rotary manner to the first portion 90 through the rotary connection 97.

The sleeve 96 is coupled with the rod-shaped element 95, for example made with a threaded rod. By varying the position of the rod-shaped element 95 engaged with the sleeve 96 the length of the third portion 94 is modified, actually modifying the inclination of the axis 83 and the value of the angle A.

As shown in the embodiment of figure 10, the sleeve 96 comprises a hollow body 98, at least one cylindrical appendix, for example a pin 99 or a similar rotary element, projecting from the sleeve 96, an abutment 100 for the rod-shaped element 95, and a safety element 101 to lock the rod-shaped element 95 together with the sleeve 96.

In the embodiment of figures 3 and 10, there are two pins 99 rotating about a third axis 102, with first brackets 103.

The first brackets 103 are fixed to the first portion 90 of the tie rod of variable length 9.

The pins 99 and the first brackets 103 actually make one of the rotary connection 97.

The abutment 100 (figure 10) is positioned passing into the opening of the hollow body 98 and is able to rotate through holding means 104.

The abutment 100 can thus rotate inside the opening of the hollow body 98 and has a threading on its inner surface corresponding to that of the rod-shaped element 95, making a screw-nut screw type coupling with the aforementioned rod-shaped element 95. Such a coupling is also locked in a stable position through the safety element 101.

The safety element 101 can be made, for example, with a nut screwed onto the rod-shaped element 95 close to the abutment element 100, thus being a lock nut as illustrated in figure 1.

A second end of the rod-shaped element 95, opposite the portion engaged with the sleeve 96, is rotatably connected to the second portion 92, through a rotary connection 97.

An electronic angular position sensor 19, suitable for measuring the angle of incidence A of the specimen 4, 400 against the abrasive surface 5, is constrained to one of the rotary connections 97.

In a further embodiment of figure 7, the specimen holder 300 can be made with a rigid body, shaped like a foot, so as to allow the abrasion test to be carried out on a specimen 400 that is shaped like a complete sole.

The specimen holder 300, indeed, makes it possible to position a shoe complete with the specimen 400 to be tested, or possibly just the specimen 400, through suitable fastening means (not illustrated), on the machine 1.

It is thus possible to carry out an abrasion test on a finished sample to better characterise the resistance to abrasion not only of the material that constitutes the sole, but also of the material in the form of tread of the sole itself.

The operation of the present invention relative to the embodiment of figure 1 is as follows.

Through the connection means 86 the specimen holder 3, 300 equipped with the specimen of material to be tested 4, 400 is connected to the second movement means 8.

The cleaning device 11 is actuated, setting in rotation the brushes 14 placed in contact with the abrasive surface 5, to prepare the surface for the next test and so as to make the surface totally free from possible residues of material due to previous tests.

The second motor means 8 are actuated to cyclically move the specimen holder 3, 300, and therefore the specimen 4, 400, against the abrasive surface 5.

The specimen 4, 400 after having come into contact with the abrasive surface 5, is pressed against the abrasive surface 5 itself by an actuator 10, with a force that can be set at the start of every test.

The first motor means 70 are actuated to cyclically move the abrasive surface 5, between two limit positions, along the first guide 73.

The movement of the specimen holder 3, 300 and of the abrasive surface 5 is shown schematically in figure 9.

The inclined line "S" represents a possible movement cycle of the mobile support base 6, and therefore of the abrasive surface 5, within the two limit positions, contained between the horizontal lines, "O" start position, and "S1" end position.

When the mobile support base 6 comes close to the end position "S1" the first end of stroke sensor 72 generates a signal, represented by the vertical line B.

Such a signal controls the return of the mobile support base 6 to the initial position "O".

The saw-tooth line "P" represents a possible movement cycle of the specimen holder 3, 300 against the abrasive surface 5.

The movement of the specimen holder 3, 300 takes place between the limit positions contained within the range of the horizontal lines "O" - the start position in which the specimen 4, 400 is pressed against the abrasive surface 5, and "P1" - the end position in which the specimen 4, 400 is not in contact with the abrasive surface 5.

When the specimen 4, 400 is pressed against the abrasive surface 5 there is rubbing due to the continuous and linear movement of the abrasive surface 5.

Such rubbing makes it possible to simulate the phenomenon of abrasion to which the materials are subjected, in particular the materials used in making shoe soles, while walking or while running.

Debris or residues of the specimen 4, 400 can be deposited on the abrasive surface 5 while the test is being carried out. Such debris are removed through the cleaning device 11, in particular by the brushes 14, allowing the friction coefficient of the abrasive surface 5 to be kept constant.

Before carrying out every abrasion test it is possible to set the angle of incidence A of the specimen 4, 400 against the abrasive surface 5 to simulate different conditions of use to which shoe soles are generally subjected.

The angle A is modified by means of the tie rod of variable length 9 connected to the second movement means 8 of the specimen holder 3, 300.

The machine 1, according to the present invention, makes it possible to carry out an abrasion test of a material, in particular of a material used in a shoe sole, or of a complete shoe sole, by simulating walking or running, taking less time to carry out a complete test.

It should also be noted that, thanks to the combined movement of the specimen holder 3, 300 and of the abrasive surface 5, the simulation of walking or of running effectively corresponds to the real situation, whereas the machine 1 is constructively much simpler than the machines of the state of the art.

Moreover, the machine 1 makes it possible to set and vary, in a simple and practical manner, different operating parameters of an abrasion test, including the angle of incidence of the specimen with respect to the abrasive surface, the force with which the specimen is placed in contact with the abrasive surface, the laws of motion of the abrasive surface, and the material of the abrasive surface itself, in order to simulate different conditions of use of the shoe sole.

Furthermore, the machine 1 makes it possible to obtain meaningful and reproducible results. Indeed, the abrasive surface 5 is kept clean, i.e. free from deposits or debris of the test material, in the area in which the contact occurs with the specimen 4, 400. Moreover, the small dimensions of the machine 1 do not require large work spaces for its installation, and to carry out an abrasion test.

The invention thus conceived can undergo numerous modifications and variants, all of which are covered by the inventive concept.

Moreover, all of the details can be replaced by other technically equivalent elements. In practice, the materials used, as well as the shapes and contingent sizes, can be whatever according to the requirements, without for this reason departing from the scope of protection of the following claims.

## Claims

1. Machine (1) for carrying out abrasion tests on materials for shoe soles or on parts of soles or on whole soles, comprising a frame (2), a specimen holder (3, 300) associated to the frame (2), at least one specimen (4, 400) fixed to the specimen holder (3, 300), an abrasive surface (5), a movable support base (6) for said abrasive surface (5), the movable base (6) being connected to the frame (2), first means for cyclically moving (7) said movable support base (6), second means for cyclically moving (8) said specimen holder (3, 300) towards said abrasive surface (5), **characterised in that** said second means for moving (8) comprise a tie rod of variable length (9) connected to said frame (2) and actuating means (10) for pushing and allowing variable inclinations of the specimen (4, 400) against the abrasive surface (5).

2. Machine according to claim 1, comprising a cleaning device (11) for said abrasive surface (5).

3. Machine according to claim 1 or 2, wherein said second means for moving (8) comprise second motor means (80), for example a motor reducer with electric servomotor, a second motion transmission device (81), and second guiding means (82) of said specimen holder (3, 300) towards said abrasive surface (5) along a second axis (83).

4. Machine according to one or more of the previous claims, wherein said second guiding means (82) comprise at least a pair of hollow supports (84), for example self-lubricating and/or sliding bushes and/or linear ball bearing or similar elements, at least one tubular element (85), passing through said hollow supports (84), and a connecting means (86) to bind said specimen holder (3, 300) to said second guiding means (82).

5. Machine according to one or more of the previous claims, wherein said actuating means (10) are linked to an end of said second means for moving (8), and at another end thereof to a connecting means (86) through an elastic joint (87).

6. Machine according to one or more of the previous claims, wherein said movable support base (6) comprises at least one fixing element (61) of said abrasive surface (5) to said first means for moving (7), and a second fixing element (75) to said frame (2).

7. Machine according to one or more of the previous claims, wherein said first means for moving (7), comprise first motor means (70), for example a motor reducer group with electric servomotor or alike, a first device of transmission of the movement (71), for example a piston with recirculating ball bearing or alike, and at least one end of stroke sensor (72) suitable for determining the reaching of a limit position of said movable support base (6) along a first guide (73).

8. Machine according to one or more of the previous claims, wherein said first means for moving (7) are connected to said movable support base (6) through a first elastic joint (74).

9. Machine according to one or more of the previous claims, wherein said fixing element (61), for example a plate bound to said movable support (6) through screws or similar elements, binds said abrasive surface (5) to said movable support base (6).

10. Machine according to one or more of the previous claims 2 to 9, wherein said cleaning device (11) comprises at least one brush (14), third motor means (15) of said brush (14) and third connecting means (16) of said cleaning device (11) to said frame (2).

11. Machine according to claim 10, wherein said brush (14) is in contact with said abrasive surface (5) and is put into rotation by said third motor means (15) with respect to a first axis (17) parallel to said abrasive surface (5).

12. Machine according to one or more of the previous claims, comprising a collecting container (18) for the debris of said specimen (4, 400), positioned on said frame (2) near said abrasive surface (5).

13. Machine according to one or more of the previous claims, wherein said variable length tie rod (9) comprises at least a first portion (90) and a second portion (92) of constant length and at least a third portion (94) of variable length.

14. Machine according to claim 13, wherein said first portion (90), said second portion (92) and said third portion (94) are connected to each other through rotary connection (97).

15. Machine according to claim 13 or 14, wherein said third portion (94) comprises a rod-shaped element (95) and at least one sleeve (96).

16. Machine according to claim 15, wherein said one sleeve (96) is pivotally bound to the first portion (90) through said rotary connection (97).

17. Machine according to claim 15 or 16, wherein said one sleeve (96) comprises a hollow body (98), at least one cylindrical appendix (99) projecting from said sleeve (96), an abutment (100) for said rod-shaped element (95), and a safety element (101), for example a nut or alike, to mutually block said rod-shaped element (95) together with said sleeve (96).

18. Machine according to claim 17, wherein said abutment (100) is pivotally bound inside said hollow body (98) suitable for realizing a screw-nut screw coupling with said rod-shaped element (95).

19. Machine according to one or more of the previous claims 13 to 18, comprising an electronic angular position sensor (19) suitable for measuring an incidence angle A of said specimen (4, 400) against said abrasive surface (5), said sensor (19) being bound to one of said rotary connection (97).

20. Machine according to one or more of the previous claims, wherein said specimen holder (300) is a rigid and/or semi-rigid body, with a foot shape, supporting a specimen (400).

21. Machine according to one or more of the previous claims, wherein said specimen (400) is shaped like parts of shoe sole and/or whole shoe sole and/or is a shoe provided with a testing sole.
